# FASCICULE DE BREVET EUROPEEN

(11) **EP 0 711 560 B1**
(45) Date de publication et mention de la délivrance du brevet: **27.06.2001**
(21) Numéro de dépôt: 95402476.6
(22) Date de dépôt: 07.11.1995
(51) Int. Cl.: A61K 31/7048, A61K 9/00, A61K 9/16

(54) **Composition pharmaceutique pour l'administration orale de flavonoides**
Oral anzuwendende Arzneizubereitung enthaltend Flavonoide
Pharmaceutical composition for the oral administration of flavonoids

(30) Priorité: 08.11.1994 FR 9413336
(43) Date de publication de la demande: 15.05.1996
(73) Titulaire: ADIR ET COMPAGNIE, 92415 Courbevoie Cédex (FR)
(72) Inventeur: Huet de Barochez, Bruno, F-45520 Chevilly (FR); Piot, Noel, F-45160 Olivet (FR); Cuine, Alain, F-45800 Saint Jean de Braye (FR)

(56) Documents cités:
- EP-A- 0 275 005
- EP-A- 0 352 190
- EP-A- 0 541 874
- WO-A-85/03865
- FR-A- 2 355 510
- FR-A- 2 577 437
- FR-A- 2 661 610
- FR-A- 2 668 705
- FR-M- 6 967
- 'Dictionnaire Vidal', 1989, EDITIONS DU VIDAL, PARIS, FR edition 65 "Daflom"
- DATABASE WPI Derwent Publications Ltd., London, GB; AN 1983-762898 & RO 81 345 A (INST. MEDICINA FARM.)

## Description

La présente invention a pour objet une nouvelle composition pharmaceutique destinée à une administration orale de flavonoïdes extraits de rutacées et plus particulièrement de diosmine.

La diosmine est une molécule utilisée dans le traitement de la maladie veineuse, comme veinotonique et vasculo-protecteur. A ce titre, elle est administrée à des doses allant de 150 à 1000 mg par prise, et ce, en 2 à 4 administrations journalières afin de traiter les manifestations de l'insuffisance veineuse chronique des membres inférieurs, fonctionnelle et organique, ainsi que pour le traitement des signes fonctionnels liés à la crise hémorroïdaire. La diosmine est habituellement administrée sous forme de comprimés dosés entre 150 et 450 mg. Une forme sachet dosée à 300 mg existe également sur le marché. La diosmine est un flavonoïde extrait de rutacées qu'il est difficile de séparer d'autres flavonoïdes. Les fractions flavonoïques purifiées utilisées dans l'invention contiennent 90 % de diosmine pure et 10 % d'autres flavonoïdes.

Des travaux récents ont montré l'intérêt d'administrer des doses élevées de diosmine. Ce nombre de doses et de prises journalières est relativement élevé du fait d'une forte métabolisation du principe actif dans le tractus gastro-intestinal. De ceci résulte une des principales difficultés d'emploi de la diosmine, à savoir une dose élevée de principe actif à administrer.

Il est donc nécessaire d'administrer des quantités importantes de diosmine par voie orale, et ce, sous une forme facile à ingérer, les traitements par la diosmine étant souvent des traitements au long cours. Or la taille des comprimés ou des gélules à avaler ne peut être augmentée au-delà d'une certaine limite. C'est pourquoi les granulés effervescents de diosmine, objets de cette invention, présentés sous forme de comprimés effervescents ou de sachets de granulé effervescent sont une alternative tout à fait intéressante. Il est facile par ce moyen d'administrer des quantités de poudre relativement importante, ces poudres peuvent être facilement dispersées dans un verre d'eau.

L'état antérieur de la technique est notamment illustrée par la demande de brevet EP 541874 qui décrit une préparation pulvérulante de diosmine, à remettre en suspension dans de l'eau au moment de l'emploi. Cette préparation contient plus particulièrement de la diosmine de granulométrie comprise entre 100 et 400 micromètres et un agent mouillant tensio-actif non-ionique.

Il est important en effet que la diosmine puisse être mouillée facilement afin de pouvoir se disperser correctement dans l'eau. Plus la dispersion est fine, plus l'administration est aisée, et mieux s'effectuera l'absorption du principe actif. En effet, la diosmine est un composé très peu soluble dans l'eau, et un des moyens d'accroître sa solubilité, est d'augmenter la surface de contact entre l'eau et la diosmine. Parmi les moyens d'accroître cette surface, le broyage et la micronisation peuvent être cités. Par micronisation, on entend habituellement une opération de réduction de la taille des particules, par exemple au moyen d'un microniseur à jet d'air, jusqu'à ce que la granulométrie moyenne des poudres se situe entre 0,1 et 10 micromètres.

Le principe retenu dans la présente invention est une micronisation de la diosmine et la présentation de cette diosmine dans un granulé effervescent.

La diosmine finement micronisée est associée à une formule de granulé effervescent. Il est apparu en effet de façon surprenante que grâce à la dispersion créée brutalement par l'effervescence, la mise en suspension de la diosmine était très facile, même si la granulométrie de cette même diosmine était très faible, ce qui est en général un obstacle à une dispersion dans l'eau des produits peu ou pas solubles dans ce solvant.

Ce procédé original évite également le recours à un agent tensioactif, produit pas toujours bien toléré par l'organisme, et qui donne en général un goût désagréable aux préparations.

La formule de l'invention décrite associe à la diosmine un acide carboxylique et un ou des carbonates pour créer l'effervescence, un agent édulcorant, et un aromatisant. Sont également inclus dans la formule des excipients pharmaceutiquement acceptables tels un colorant, de manière à donner un aspect agréable à la suspension reconstituée (la diosmine possède une couleur naturelle peu agréable à l'oeil), comme par exemple le jaune orangé S, un édulcorant de synthèse, de manière à diminuer la quantité de sucre utilisée, comme par exemple le saccharinate de sodium, l'aspartam ou la néohespéridine dihydrochalcone, un composé destiné à limiter la mousse formée par le couple effervescent, comme par exemple la polyvidone ou une maltodextrine, des lubrifiants, comme par exemple le stéarate de magnésium ou le benzoate de sodium, des agents d'écoulement, comme par exemple la silice colloïdale anhydre.

D'autre part, le procédé de préparation de ces granulés effervescents, objet également de l'invention, présente une caractéristique tout à fait surprenante. En effet, la diosmine est un produit hygroscopique qui contient en général entre 4 et 6 % d'eau. Cette eau constitue une gène au niveau de la stabilité du granulé effervescent en ce qu'elle peut provoquer l'effervescence spontanée du granulé. L'utilisation simultanée de bicarbonate et de carbonate de sodium permet de piéger une partie de l'humidité interne du granulé, mais n'est pas suffisante pour conduire à une bonne stabilité.

Une des possibilités habituellement utilisée pour éviter cet inconvénient, est d'avoir recours à une double granulation, à savoir une séparation de l'acide carboxylique et du bicarbonate de sodium dans deux granulés différents, réunis en final dans le sachet. Ceci est une forte contrainte au niveau de la production industrielle de tels sachets.

Le procédé de fabrication selon l'invention est un procédé monophasique original. Il consiste à utiliser l'eau interne de la diosmine pour débuter la granulation, cette eau interne provoquant un début d'effervescence qui est contrôlée pendant la phase de granulation. L'ajout d'une quantité minimale d'alcool éthylique permet de terminer la granulation. Après séchage, l'humidité finale du granulé est inférieure à 0,5 %. Dans ces conditions, le granulé effervescent est stable.

Ce granulé peut être ensuite réparti en sachet ou peut être comprimé.

Ainsi, la composition pharmaceutique selon l'invention est un granulé effervescent présenté sous forme de comprimés ou de sachets permettant une remise en suspension fine de diosmine micronisée, dont le diamètre moyen est compris entre 0,1 et 10 micromètres. Cette composition pharmaceutique ne contient pas d'agent tensioactif ni d'agent épaississant.

La composition pharmaceutique selon l'invention contient un agent édulcorant qui peut être du saccharose ou un autre sucre tel le mannitol ou le lactose.

Le procédé de fabrication de la composition pharmaceutique selon l'invention est un procédé de granulation monophasique utilisant l'eau interne de la diosmine pour débuter la granulation.

Enfin, les compositions pharmaceutiques ainsi réalisées contiennent entre 0,1 et 5 g de diosmine micronisée par prise unitaire.

Les exemples suivants illustrent l'invention sans toutefois la limiter.

### EXEMPLE 1 : Formule de granulé effervescent au saccharose

| **Constituants** | **Quantités (mg)** |
|---|---|
| Fraction flavonoïque purifiée micronisée comprenant : Diosmine 90 % Flavonoïdes exprimés en hespéridine 10 % | 1000 |
| Acide citrique anhydre | 570 |
| Arôme orange | 150 |
| Carbonate de sodium anhydre | 50 |
| Bicarbonate de sodium | 450 |
| Jaune orangé S | 3 |
| Saccharinate de sodium | 2 |
| Saccharose | 2715 |
| Silice colloïdale hydrophobe | 20 |
| | |
| **Pour un sachet terminé à** | **5000 mg** |

### EXEMPLE 2 : Formule de granulé effervescent au mannitol

| **Constituants** | **Quantités (mg)** |
|---|---|
| Fraction flavonoïque purifiée micronisée comprenant : Diosmine 90 % Flavonoïdes exprimés en hespéridine 10 % | 1000 |
| Acide citrique anhydre | 570 |
| Arôme orange | 150 |
| Bicarbonate de sodium | 450 |
| Carbonate de sodium anhydre | 50 |
| Jaune orangé S | 3 |
| Mannitol | 2461 |
| Polyvidone | 250 |
| Saccharinate de sodium | 6 |
| Silice colloïdale hydrophobe | 20 |
| | |
| **Pour un sachet terminé à** | **5000 mg** |

### EXEMPLE 3 : Formule de granulé effervescent au lactose

| **Constituants** | **Quantités (mg)** |
|---|---|
| Fraction flavonoïque purifiée micronisée comprenant : Diosmine 90 % Flavonoïdes exprimés en hespéridine 10 % | 1000 |
| Acide citrique anhydre | 570 |
| Arôme orange | 150 |
| Bicarbonate de sodium | 450 |
| Carbonate de sodium anhydre | 50 |
| Jaune orangé S | 3 |
| Lactose | 2332 |
| Polyvidone | 375 |
| Saccharinate de sodium | 10 |
| Silice colloïdale hydrophobe | 20 |
| | |
| **Pour un sachet terminé à** | **5000 mg** |

### EXEMPLE 4 : Formule de comprimé effervescent

| **Constituants** | **Quantités (mg)** |
|---|---|
| Fraction flavonoïque purifiée micronisée comprenant : Diosmine 90 % Flavonoïdes exprimés en hespéridine 10 % | 1000 |
| Acide citrique anhydre | 600 |
| Arôme orange | 60 |
| Benzoate de sodium | 15 |
| Bicarbonate de sodium | 450 |
| Carbonate de sodium anhydre | 50 |
| Jaune orangé S | 1 |
| Polyvidone | 100 |
| Saccharinate de sodium | 1 |
| Sorbitol | 723 |
| | |
| **Pour un sachet terminé à** | **3000 mg** |

## Revendications

1. Composition pharmaceutique pour l'administration orale de flavonoïdes extraits de rutacées et plus particulièrement de diosmine caractérisée en ce que cette composition est un granulé effervescent présenté sous forme de comprimé ou de sachet contenant de la diosmine micronisée.

2. Composition pharmaceutique selon la revendication 1 caractérisée en ce que la diosmine sous forme micronisée présente une granulométrie comprise entre 0,1 et 10 micromètres.

3. Composition pharmaceutique selon la revendication 1 caractérisée en ce que le granulé est rendu effervescent par l'utilisation d'un acide carboxylique et d'un ou de plusieurs carbonates.

4. Composition pharmaceutique selon la revendication 1 caractérisée en ce que le granulé effervescent contient un agent édulcorant, un aromatisant et des excipients pharmaceutiquement acceptables.

5. Composition pharmaceutique selon la revendication 1 caractérisée en ce qu'elle contient de 0,1 à 5 g de diosmine micronisée.

6. Procédé de préparation de la composition pharmaceutique selon la revendication 1 caractérisée en ce qu'il s'agit d'un procédé de granulation monophasique qui utilise les 4 à 6 % d'eau contenu dans la diosmine pour débuter la granulation.

## Patentansprüche

1. Pharmazeutische Zubereitung zur oralen Verabreichung von aus Rutaceen extrahierten Flavonoiden und insbesondere von Diosmin, dadurch gekennzeichnet, daß diese Zubereitung ein aufschäumbares Granulat ist, welches in Form einer das mikronisierte Diosmin enthaltenden Tablette oder Sachets vorliegt.

2. Pharmazeutische Zubereitung nach Anspruch 1, dadurch gekennzeichnet, daß das in mikronisierter Form vorliegende Diosmin eine Teilchengröße zwischen 0,1 und 10 *µ*m aufweist.

3. Pharmazeutische Zubereitung nach Anspruch 1, dadurch gekennzeichnet, daß das Granulat durch Verwendung einer Carbonsäure und eines oder mehrerer Carbonate aufschäumbar ausgebildet ist.

4. Pharmazeutische Zubereitung nach Anspruch 1, dadurch gekennzeichnet, daß das aufschäumbare Granulat ein Süßungsmittel, ein Aromatisierungsmittel und pharmazeutisch annehmbare Bindemittel enthält.

5. Pharmazeutische Zubereitung nach Anspruch 1, dadurch gekennzeichnet, daß sie 0,1 bis 5 g mikronisiertes Diosmin enthält.

6. Verfahren zur Herstellung der pharmazeutischen Zubereitung nach Anspruch 1, gekennzeichnet durch ein einphasiges Granulationsverfahren, welches zur Auslösung der Granulation die in dem Diosmin enthaltenen 4 bis 6 % Wasser ausnützt.

## Claims

1. Pharmaceutical composition for the oral administration of flavonoids extracted from Rutaceae and more especially of diosmin, characterised in that that composition is an effervescent granulate in the form of a tablet or a sachet comprising micronised diosmin.

2. Pharmaceutical composition according to claim 1, characterised in that the diosmin in micronised form has a granule size of from 0.1 to 10 micrometres.

3. Pharmaceutical composition according to claim 1, characterised in that the granulate is rendered effervescent by using a carboxylic acid and one or more carbonates.

4. Pharmaceutical composition according to claim 1, characterised in that the effervescent granulate comprises a sweetener, a flavouring and pharmaceutically acceptable excipients.

5. Pharmaceutical composition according to claim 1, characterised in that it comprises from 0.1 to 5 g of micronised diosmin.

6. Process for the preparation of the pharmaceutical composition according to claim 1, characterised in that it is a single-phase granulation process using the from 4 to 6% water contained in diosmin to start the granulation.
